# EUROPEAN PATENT APPLICATION

(11) **EP 3 372 145 A1**
(43) Date of publication of application: **12.09.2018**
(21) Application number: 17159967.3
(22) Date of filing: 08.03.2017
(51) Int. Cl.: A61B 3/12, A61B 5/026, G01F 1/66

(54) **METHOD FOR PROCESSING AN ELECTRICAL SIGNAL PROVIDED BY A LASER DOPPLER FLOWMETER AND A LASER DOPPLER FLOWMETER**

(71) Applicant: Y-Ybar Sarl, 1950 Sion (CH)
(72) Inventor: GEISER, Martial, 1950 Sion (CH); TRUFFER, Frederic, 3928 Randa (CH); MARAZZI, Francesco, 41049 Sassuolo (MO) (IT)
(74) Representative: Gevers SA

(57) **Abstract**

The invention provides a method for processing an electrical signal provided by a laser Doppler flowmeter. The method comprises the steps of:
- Providing a laser Doppler flowmeter signal and separating the DC and AC components of the signal so as to provide a DC signal and an AC signal;
- Amplifying the AC component by a first amplification and the DC component by a second amplification factor to obtain separated amplified AC and DC signals;
- Generating a weighted signal from a variable offset electrical signal, the amplified AC component and the amplified DC component;
- Filtering the weighted signal and converting it with an A/D converter and processing the converted signal with computing means to obtain a laser Doppler signal proportional to the liquid flow-

The invention is also achieved by a laser Doppler flowmeter using the method of the invention.

## Description

### Technical Field

The present invention relates to the field of laser Doppler flowmetry and laser Doppler velocimetry. More particularly, the present invention relates to a method for processing an optical signal provided by a laser Doppler flowmeter or velocimeter comprising a detector and electronic means to collect and measure backscattered light comprising a DC and an AC signal component.

### State of the art

Laser Doppler flowmetry (LDF) and laser Doppler velocimetry (LDV) are non-invasive techniques allowing monitoring microvascular blood flow and blood velocity in vessels in research and in clinical routines.

LDF can be applied to the eye to study the blood flow in the optic nerve head, the retinal microcirculation as well as the choriocapillaris in the foveal region of the choroid, which may become important in the investigation of the pathogenesis of age-related macular degenerations. Another application is in the evaluation of subfoveal choroidal blood flow in macular degenerescence or in high myopic eyes indicted by changes such as dynamic and isometric exercises or drugs.

With the same principle, measurement of velocity in blood vessels can be done (laser Doppler velocimetry).

These applications are described in the following publications:
- C. E. Riva, "Laser Doppler Techniques for Ocular Blood Velocity and Flow," in Ocular Blood Flow, no. 7, Berlin, Heidelberg: Springer Berlin Heidelberg, 2012, pp. 123-146.
- C. J. Pournaras and C. E. Riva, "Retinal blood flow evaluation.," Ophthalmologica, vol. 229, no. 2, pp. 61-74, 2013.
- C. E. Riva, "Ocular blood flow assessment using continuous laser Doppler flowmetry.," vol. 88, no. 6, pp. 622-629, Sep. 2010.
- C.E.Riva et al:"Laser Doppler flowmetry in the optic nerve head", Exp.Eye Res.55, 499506, 1992;
- C.E.Riva et al: " Choroidal blood flow in the foveal region of the human ocular fundus", Invest.Opthalmol, Visual Sci 35, 42734282, 1994;
- J.E.Grinwald et al." Effect of aging on foveolar choroidal circulation", Arch Opthalmol (Chicago), 116, 150154, 1998;
- A. Friedman. " A hemodynamic model of the pathogenesis of age-related macular degeneration", Am.J.Opthalmol. 125, 677682, 1997.

Laser Doppler flowmetry techniques can also be used in dermatology with applications in the field of pharmacology to study the microvascular effects of vasoactive substances and drugs, in plastic surgery or to study changes in microvascular blood flow in diabetic patients or for people having peripheral vascular diseases linked to the Raynaud's phenomenon.

The following publications treat extensively these possible applications:
- I. Fredriksson, M. Larsson, and T. Stromberg, "Laser Doppler Flowmetry," in Microcirculation Imaging, M. Leahy, Ed. 2012, pp. 67-87.
- Fredriksson et al. "Model-based quantitative laser Doppler flowmetry in skin.," vol. 15, no. 5, p. 057002, Sep. 2010.
- Humeau, Anne et al.."Spectral components of laser Doppler flowmetry signals recorded in healthy and type 1 diabetic subjects: scalogram analyses", physics in medicine and biology 49.17, 2004, p.3957
- G.Michelson et al." Clinical investigation of the combination of a scanning layer opthalmoscope and laser Doppler flowmeter", Ger.J.Opthlamology 4, 342349, 1995;
- Pemp, berthold et al. "Strategies for reducing variance in laser Doppler flowmetry measurements, vol.247, nr 1, pp.6771, 2009;

LDF or LDV is based on coherent light that is focused on a partially transparent channel that comprises a circulating liquid, for example a tissue comprising blood channels. When a coherent light is focused on a tissue a part of this light will be partially absorbed and partially scattered by moving red blood celles (RBC). The backscattered light will be Doppler shifted as described in:
- R.Bonner et al. "Model for laser Doppler measurements of blood flow in tissue", applied Optics, 15 June 1981, bvol.20, nr.20;
- C. Riva, B. Ross, and G. B. Benedek, "Laser Doppler measurements of blood flow in capillary tubes and retinal arteries.," Invest Ophthalmol, vol. 11, no. 11, pp. 936-944, Nov. 1972.

The backscattered light comprises a summation of light waves having various frequncy shifts Δfs proportional to the scatter velocity and the non-shifted frequency light beam. By optically collecting on a detector the beating between all these optical waves, information about blood perfusion can be extracted as described in:
- -L.B.Petrig and C.E.Riva:" Nerve Head Laser Doppler Flowmetry: principles and Computer Analyses, in Ocular Blood flow, S.Karger AG, 1996, pp.120-127.
- C.E.Riva et al. "Bidirectional LDV system for absolute measurement of blood speed in retinal vessels," vol. 18, no. 13, pp. 2301-2306, 1979.

The extraction of information is done by realizing a Doppler shifted power spectrum (DSPS).

The computation of the blood perfusion parameters for LDF are:
- The DC value which is important to the intensity of the detected light;
- The first moment of the DSPS (Vel) which is proportional to the mean velocities of the RBC's;
- The power of the DSPS normalized with the DC (Vol), which is assumed to be proportional to the number of scatters that occur within a probing volume;
- In addition, the computation of the velocity is done by estimating the cut-off frequency of the DSPS.

The computation of the blood velocity for LDF and LDV is usually done by finding the cut-off frequency of said DSPS.

To get a reliable DSPS and to reduce the variance of the measurements it is important to detect the signal with a high amplitude resolution (usually 12 to 16 bits) and a constant amount of backscattered light (DC). The useful information can be extracted typically by the portion of the spectra between 30Hz and 40KHz, so a sampling rate of at least 80kHz should be used, in agreement with the Nyquist-Shannon sampling theorem.

A typical laser Doppler flow system of prior art. is described in: Geiser et al. "Compact laser Doppler choroidal flowmeter", Journal of Biotechnical optics, 4.4, 1999, p.459-464. In the system described by Geiser et al., and in all LDF systems of prior art, the AC part of the signal is very small compared to the DC level and severe quantization errors occur which limits the precision and reliability of the system. The system described by Geiser et al. also requires the use of a computer which makes the system bulky and limits the miniaturization possibilities as well as the number of possible parallel LDF acquisitions.

### Summary of the invention

The object of the invention is to provide an improved method for processing an electrical signal provided by a laser Doppler flowmeter or a laser Doppler velocimeter. The method solves the limitations of known processing methods of an optical signal provided by a laser Doppler flowmeter.

More precisely the method of the invention comprises the steps of:
- Detecting at least a portion of a light beam scattered by a flowing liquid upon which a coherent light beam from a laser Doppler flowmeter impinges with a detector;
- Generating an electrical signal proportional to said detected portion of the scattered light beam;
- Separating the DC and AC components of said electrical signal so as to provide a DC signal and an AC signal;
- Amplifying the AC component by a first amplification and the DC component by a second amplification factor to obtain amplified AC and DC signals such that an absolute value of said amplified AC component, expressed in voltage, is preferably comprised between 0.5 and 100 times an absolute value, expressed in voltage, of said amplified DC component,
- Providing a variable offset electrical signal,
- Generating a weighted signal from said variable offset electrical signal, said amplified AC component and said amplified DC component;
- Filtering said weighted signal;
- Converting said filtered signal with an A/D converter and processing the converted signal with computing means to obtain a laser Doppler signal proportional to the liquid flow, said laser Doppler signal being such that analyzing its frequency spectrum it is possible to reconstruct the volume and the speed of the flow'.

In an embodiment said amplified AC component has a value, expressed in voltage, between 0.5 and 50 times the value, expressed in voltage, of said amplified DC component.

In an embodiment said amplified AC component has a value, expressed in voltage, between 1 and 25 times the value, expressed in voltage, of said amplified DC component.

In an embodiment said flowing liquid is a liquid flowing in a partially transparent tube.

In an embodiment said tube is an artery or a vein and said liquid is liquid blood.

In an embodiment said tube is a mesh of fluid capillaries of a living tissue.

In an embodiment said fluid capillaries are blood capillaries.

The invention is also achieved by a laser Doppler flowmeter or velocimeter comprising a laser to send, in operation, a light beam onto a flowing liquid, and comprising optical elements to collect, in operation, backscattered light from said flowing liquid, further comprising a laser Doppler flowmetry acquisition system comprising a detector to collect said backscattered light comprising a DC and an AC signal component.

The laser Doppler flowmetry acquisition system comprises:
- means to separate the DC and AC signal component of said electrical signal so as to provide a DC signal and an AC signal;
- first amplification means to amplify said AC signal by a first amplification factor so as to obtain an amplified AC signal;
- second amplification means arranged to amplify said DC signal component by a second amplification factor so as to obtain an amplified DC signal and so that said amplified AC signal has a value, expressed in voltage, preferably between 0.5 and 100 times the value, expressed in voltage, of said amplified DC signal
- means arranged to provide a variable offset signal;
- a weighted summing amplifier arranged to input said variable offset, said amplified AC and said amplified DC component, so as to obtain, in operation, a weighted signal;
- a low-pass filter arranged to input said weighted signal, to provide a filtered signal;
- an A/D converter arranged to input said filtered signal, to provide a converted signal.

In an embodiment, said first amplification factor is between 0.5 and 50.

In an embodiment, said first amplification factor is between 1 and 25.

In an embodiment, said laser Doppler flowmetry acquisition system comprises an electronic circuit to perform a Fourier transform on said converted signal so as to provide a laser Doppler signal proportional to the flow of said liquid.

In an embodiment, said laser Doppler flowmetry acquisition system comprises computing means to process further the laser Doppler signal.

The invention is also achieved by the use of a laser Doppler flowmeter as described before for measuring blood flow in a blood vessel situated in the human eye.

### Brief description of the drawings

Further details of the invention will appear more clearly upon reading the following description in reference to the appended figures:
- Fig. 1 shows a general view of a laser Doppler flowmetry system of prior art;
- Fig. 2a-2b illustrate the steps for processing an electrical signal provided by a laser Doppler flowmeter;
- Fig.3 illustrates a power spectrum obtained by a rotating wheel and a power spectrum obtained by a human eye as Doppler generators;
- Fig.4 illustrates the steps of the separation of an AC and a DC signal, their amplification, and their addition to an offset signal by a summing amplifier of the laser Doppler flowmetry system of the invention;
- Fig.5 illustrates a Doppler signal in function of the rotation rate of a light scattering diffuser wheel obtained by a Doppler flowmeter of the invention;
- Fig.6 shows a statistical distribution of a measurement performed by a flowmeter of prior art and by a Doppler flowmeter of the invention;

### Embodiments of the invention

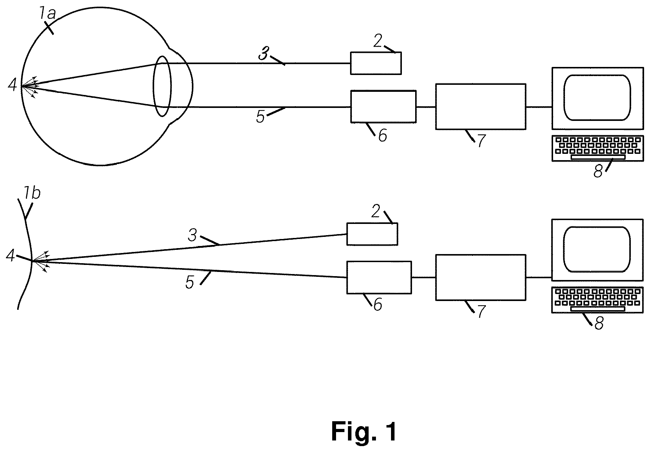

Fig.1 illustrates a laser Doppler flowmeter of prior art as used in the case of a human eye 1 a or human skin 1 b. Such a laser Doppler flowmeter comprises a laser or coherent light source 2 emitting a laser beam 3. This laser beam 3 provides a scattered light beam 4. This scattered light consists of a summation of waves having various frequency shifts which are proportional to the velocity of the scattering elements, which are mainly red blood cells, in the light beam and the portion of scattered light which is not shifted in frequency.

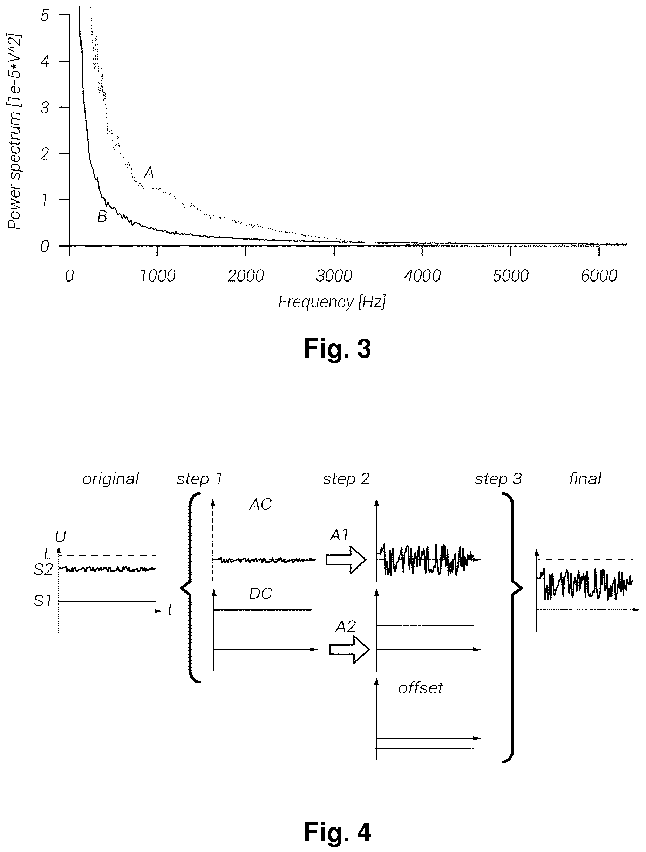

Fig.3 shows a comparison between the power spectrum obtained using a rotating light scattering wheel, also called diffuser, and a human eye as Doppler signal generators.

In a laser Doppler flowmeter of prior art a portion of the scattered light beam 4 is collected as a collected scattered beam portion 5 and is detected by a detector 6 that is adapted to detect the collected portion 5. The detector 6 is configured to convert the detected light beam into an analog electrical signal. The analog electrical signal is converted into a digital signal by an analog to digital converter 7. This digital signal is preferably provided to a computer 8.

By optically collecting, by a detector 6, the beating of all the detected optical waves, information on the flow of the liquid may be obtained, such as the blood perfusion. The extraction of information is preferably done through the realisation of a Doppler shift power spectrum (DSPS).

In an example of application the blood perfusion parameters may be computed. These parameters are mainly:
The DC value of the detected signal, which is proportional to the intensity of the detected light;
The first moment of said DSPS, defined as Vel, which is proportional to the mean velocities of the red blood cells;
The power of the DSPS, normalized with the square of the DC signal (DC²), defining a normalized signal Vol, which is assumed to be proportional to the number of light scattering sources, also defined as light scatterers, in the probing volume of the laser Doppler system.

In order to get a reliable DSPS and to reduce the variance of the measurements, it is important to detect the signal with a high amplitude resolution and a substantially constant amount of backscattered light, corresponding to a DC signal. Said high resolution is preferably between 12 to 16 bits. The useful information can be extracted by the portion of the optical signal between 30 Hz and 40kHz, so that a sampling rate of at least 80kHz has to be used, in agreement with the Nyquist-Shannon sampling theorem. In order to assure a safe detection margin, the sampling rate is usually in the range of 120kHz.

The main problem with laser Doppler detection methods of prior art is that when the AC part of the detected signal is very small compared to the DC part of the optical signal, severe quantification errors occur, which leads to severe errors in the measured flow. Also, laser Doppler systems of prior art suffer from parasitic noise signals such as provided by, for example, internal optical reflections or parasitic light sources situated outside the system, or also by parasitic electronic noise signals which lead to less reliable and imprecise liquid flow measurements.

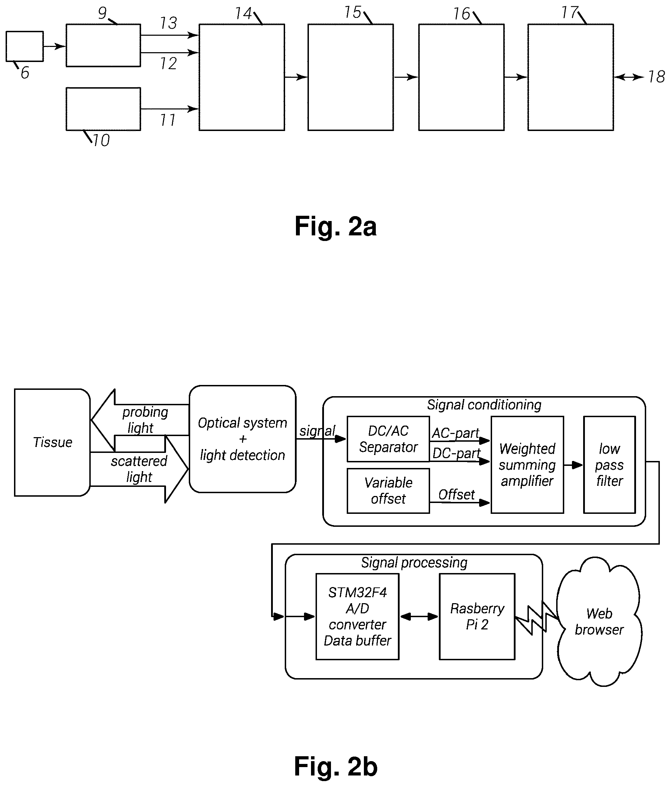

The invention is achieved by a method for processing an electrical signal provided by a laser Doppler flowmeter. The method is illustrated schematically in Figs 2a-2b, and comprises the following steps:
1. A detector 6 detects at least a portion of a light beam scattered by a flowing scattering liquid upon which a coherent light beam from a laser Doppler flowmeter impinges with a detector.
2. An electrical signal 6a proportional to said detected portion of the scattered light beam is generated.
3. The DC and AC components of said electrical signal 6a are separated by an electrical separator 9 so as to provide a DC signal 12 and an AC signal 13. This separation allows to process the AC and DC signal with different electrical circuits as illustrated in Fig.2b.
4. The AC signal component 13 is amplified by a first amplification factor, providing an amplified AC component 13a and the DC signal 12 component is amplified by a second amplification factor, providing an amplified DC signal 12a so as to obtain amplified AC and DC signals such that an absolute value of said amplified AC component, expressed in voltage, is preferably comprised between 0.5 and 100 times an absolute value, expressed in voltage, of said amplified DC component; The amplified AC component may also be comprised between 0.5 and 50, preferably between 1 and 25 times said absolute value.
5. A variable offset electrical signal 11 is provided by an electric circuit 10.
6. A weighted signal 14a from said variable offset electrical signal, said amplified AC component and said amplified DC component is provided by a weighted summing amplifier 14. Otherwise said, the weighted summing amplifier provides a sum of an adapted offset electrical signal, an amplified AC component and an adapted DC component.
7. Said weighted signal 14a is filtered by an electronic filter 15 providing a filtered electrical signal 15a.
8. Said filtered signal 15a is converted with an A/D converter 16 providing a converted electrical signal 16a.
9. The converted signal 16a is processed with computing means 17 to obtain a laser Doppler signal proportional to the liquid flow.

The steps of the separation of said AC and DC signals, their amplification and the adding to an offset signal is illustrated schematically in Fig. 4. In Fig.4 the original signal means the signal obtained by a laser Doppler system before the processing according to the method of the invention and the final signal means the processed electrical signal obtained by according to the electrical processing method of the invention. In Fig. 4 the first and second amplification factors are illustrated by factors A1 and A2.

It is generally admitted that the DC component of the detected electrical signal, proportional to the detected optical signal, is either a positive voltage or a negative voltage depending on the application. Typical maximum values of the absolute value of the voltage of the DC component is 1 Volt. In general the AC component of the detected electrical signal has a RMS (root mean square) value in the range of millivolts, typically between 0.5 mV and 10 mV. In most embodiments the electrical processing stages require positive signals, so it is preferable to implement a voltage sign inversion scheme after the detection of the DC and the AC signals.

It is understood that the electrical processing circuit comprises electrical modules allowing the user to adapt the voltages of the AC and/or DC and/or the variable offset. Said modules comprise preferably a variable resistor. Such circuits are well known to the person skilled in the art and are not commented here.

The gain, or amplification, of the AC signal may be realized by a variable resistor. It is understood that as well the AC signal, the DC signal and the provided offset signal may each be adapted by different amplification factors in function of the application of the Laser Doppler system.

Preferably, as illustrated in Fig.2b, the filter unit 15 to which the weighted signal 14a is provided is a low-pass filter.

In an embodiment said low-pass filter is a 10^{th} order low-pass digital filter having a cut-off frequency of 43 kHz, and providing a first filtered signal. In an embodiment said low pass filter comprises a passive lowpass filter having a cutoff frequency of 100 kHz. The input of said passive filter is the first filtered signal provided by said 10^{th} order low-pass digital filter passive lowpass filter. The passive filter is intended to reduce the effect of clock feedforward effects.

The advantage of the signal processing method of the invention, as illustrated in Figs 2a-2b relates mainly to two effects:
- it allows the user to cancel the effects of background noise by providing to the signal processing electronics less DC offset in situations wherein the background noise is strong;
- the DC offset, electrically added to the amplified AC signal component allows to the analog-to-digital (A/D) converter to be used in its full range (typically between 0-3.3V) with any expected optical signal.

There are different possible variants to process said converted signal 16a. In a preferred embodiment said converted signal 16a is processed with computing means 17 comprising a microcontroller unit (MCU). For example an MCU of the type STM32F407 comprising a Direct Memory Access (DMA). This MCU samples the converted signal 16a, defined as the conditioned signal 16a, and stores the digital converted signal value in a buffer unit defined as a buffer. This buffer is required in the case of an embedded microcomputer having no real-time operating unit, such as currently commercially available systems, for example a commercial Raspberry PI microcomputer (www.raspberrypi.org), so that data are transferred to the microcomputer at a higher rate than the sampling frequency

In an exemplary realization the microcomputer, such as a Raspberry Pi, saves the processed signal in a data file, which may be locally processed or retrieved via an Ethernet network. Said data file is preferably a Comma Separated Value (CVS) file. In an exemplary configuration the commands to start and stop measurements may be sent by an Ethernet network.

It is understood that a wide variety of parameters may be adapted in the processing system, depending on the application, such as signal adaptation to correct biomedical signal artefacts as further described.

In an embodiment, said amplified AC component 13a has a value, expressed in voltage, preferably between 0.5 and 100 times the value, expressed in voltage, of said amplified DC component 12a. In an embodiment, said amplified AC component 13a has a value, expressed in voltage, between 0.5 and 50 times, preferably between 1 and 25 times the value, expressed in voltage, of said amplified DC component 12a.

In an embodiment, said amplified DC component 13a has a value, expressed in voltage, between 0.8 and 1.2 times the value, expressed in voltage, of said amplified AC component 12a.

In various embodiments:
- said flowing liquid is a liquid flowing in a partially transparent tube;
- said tube is an artery and said liquid is liquid blood;
- said tube is a mesh of fluid capillaries of a living tissue;
- said fluid capillaries are blood capillaries.

It is understood that the signal processing method as illustrated in Figs. 2a-2b may be adapted for particular situations in biomedical measurements. For example in the case of measurements into locations in the eye, eye blinking provides parasitic signals that should be reduced considerably. Similar, in the case of measurements of blood flow near to the skin, skin movements will also induce signal artefacts.

In order to reduce considerably such parasitic signals different additional steps may be implemented such as:
- discarding DC values whose absolute values are not within 20% of the mean DC signal values, in order to provide a stable signal;
- discarding Vel signal values if the DSPS signal around 100 Hz is 10 times lower than the DSPS around 30kHz, in order to ensure a meaningful Doppler signal which may be used for measuring the bloodflow;
- discarding Vol signal values upon detection of a sudden change larger than a previous value, occurring 1/15^{th} of a second before the measurement.

It is understood that these adaptations to the method are chosen based on statistical estimations provided by experiments in the specific cases of the applications.

The invention is also achieved by a laser Doppler flowmeter. The laser Doppler flowmeter comprises a laser to send, in operation, a light beam onto a flowing liquid, and comprises optical elements to collect, in operation, backscattered light from said flowing liquid. The laser Doppler flowmeter of the invention further comprises a laser Doppler flowmetry acquisition system comprising a detector to collect said backscattered light comprising a DC and an AC signal component.

In a preferred embodiment the laser Doppler flowmetry acquisition system comprises:
- means to separate the DC and AC signal component of said electrical signal so as to provide a DC signal and an AC signal;
- first amplification means to amplify said AC signal by a first amplification factor so as to obtain an amplified AC signal;
- second amplification means arranged to amplify said DC signal component by a second amplification factor so as to obtain an amplified DC signal and so that said amplified AC signal has a value, expressed in voltage, preferably between 0.5 and 100 times the value, expressed in voltage, of said amplified DC signal;
- means arranged to provide a variable offset signal;
- a weighted summing amplifier arranged to have said variable offset, said amplified AC and said amplified DC component as input, and arranged so as to obtain, in operation, a weighted signal,
- a low-pass filter arranged to input said weighted signal, to provide a filtered signal;
- an A/D converter arranged to input said filtered signal, to provide a converted signal.

In an embodiment said first amplification factor is between 0.5 and 50. In a variant said first amplification factor is between 1 and 25. Said first amplification means are preferably realized by standard electronic components.

Said second amplification means are preferably realized by standard electronic components. Said variable offset signal may be realized by standard electronic components.

In an embodiment of the laser Doppler flowmeter the amplified AC signal has a value, expressed in voltage, preferably between 0.5 and 100 times the value, expressed in voltage, of said amplified DC signal. In a variant said amplified AC signal is between 0.5 and 50 times the value, expressed in voltage, of said amplified DC signal. In another variant said amplified AC signal is between 1 and 25 times the value, expressed in voltage, of said amplified DC signal.

The weighted summing amplifier, the low-pass filter and the A/D converter may be realized by standard electronic components.

In an embodiment, said laser Doppler flowmetry acquisition system comprises an electronic circuit to perform a Fourier transform on said converted signal so as to provide a laser Doppler signal proportional to the flow of said liquid.

In an embodiment said laser Doppler flowmetry acquisition system comprises computing means to process further the laser Doppler signal.

### Experimental results

In order to validate the method and system of the invention, a laser Doppler flowmeter of prior art has been adapted in order to test the improvement of the system provided by the method of the invention.

More precisely the laser Doppler flowmeter of prior art is the one described in : M. Geiser et al. "Compact Laser Doppler Choroidal Flowmeter", Journal of Biomedical Optics, 4.4, 1999, pp.459-464 (M.Geiser hereafter), incorporated in its entirety hereafter. The flowmeter described in M.Geiser is called the reference prior art system hereafter.

To compare the experimental results obtained with the method and system of the invention with the one obtained with said reference prior art system, the same experimental procedure has been performed as described in M. Geiser.

### First experiment using a rotating wheel

A first comparison measurement setup used a rotating diffusing wheel has been used. This wheel is used as a Doppler shift generator.

The optical probing beam of the reference prior art system was focused on a rotating Teflon wheel having a diameter of 45 mm by using a 20mm focal length lens. The backscattered signal of the rotating disc exhibits a Doppler shift which is qualitatively similar, i.e. has the same type of response, to the backscattered signal produced when focusing into a human eye. The curve A of Fig. 3 shows a typical measurement result obtained by a rotating scattering disc. The curve B of Fig. 3 shows a typical measurement result obtained in the case of a human eye.

In a setup based on a rotating diffusing disc, the mean frequency shift (Vel) is expected to be proportional to the speed of the wheel. Also, because the probing volume does not change and the number of scatter sources is constant (the material is homogeneous Teflon), Vol is expected to remain constant.

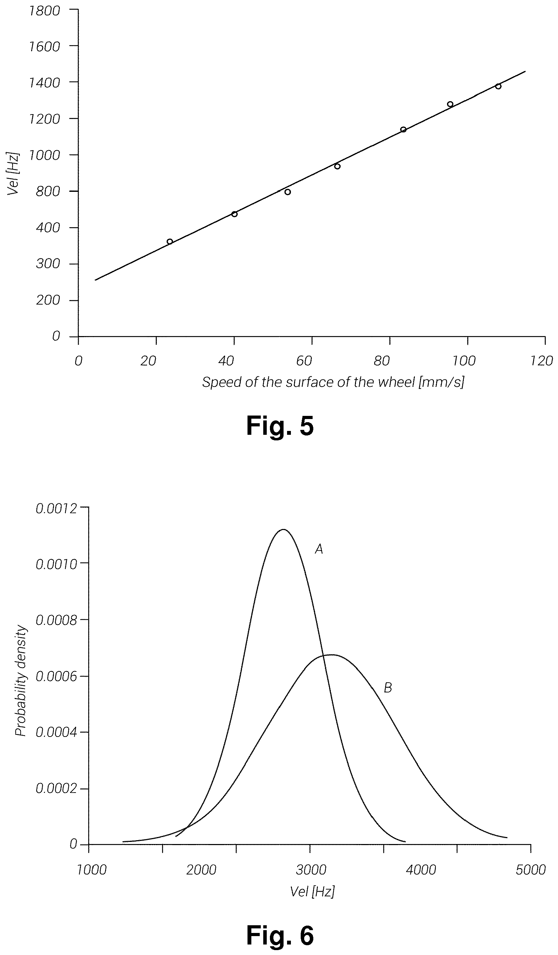

Fig. 5 illustrates the linear response obtained by the system of the invention: the obtained Doppler signal is linearly proportional to the speed of the scattering disc.

### Second experiment on the choroid of a human eye

Several volunteers have been subjected to measurements with the prior art system and the system of the invention. These experiments have been performed according to the thenets of the Declaration of Helsinki for human experimentation and in particular, the pupils of the eyes have not been dilated. In the experiments the volunteers had to look with one eye at a faint red light source (probing beam) during a 30 second recording.

Fig. 6 shows that the standard deviation and the coefficient of variation of the system of the invention, for any given recording, are smaller compared to said system of prior art. These results show that the system of the invention the system of the invention has surprising superior performances due to the simple electronic scheme of the invention as described before. No system in the prior art has addressed the problem of improving the performances of a laser Doppler system by modifying the signal processing electronics in a simple, cheap and reliable way. Additionally the system of the invention allows to provide a more compact laser Doppler system.

The comparison between the system of prior art and the system of the invention, after removing an offset difference, has shown that the system of the invention is more reliable and has a higher accuracy.

The fact that the method of the invention provides a lower standard deviation compared to the system of prior art, as illustrated in Fig.6., is due to the considerable reduction of the overall noise. Also, in addition, the fine-tuning of the thresholds and the parameters contribute to the improvement of the systems of prior art.

The possibility to filter out unwanted parts or parasitic portions of the signal play also a role in the improvement of the system. This would not be possible by systems of prior art, as in these systems the AC portion of the detected signal is not separated from the DC portion, which is essential in the method and system of the invention.

Besides the reduction in quantization noise, the system of the invention does not require having a laptop computer and its power supply close to the laser as in measurement systems of prior art. This aspect of the invention in itself contributes considerably to the reduction of noise.

Another aspect of the inventions is that it allows providing a much cheaper instrument than systems of prior art. The cost may be typically 10 times lower. The main reason of the reduction in cost is the possibility to use a microcontroller and an embedded microcomputer instead of an ADC commercial board.

In conclusion, the invention provides a laser Doppler flowmeter method and a laser Doppler flowmeter in which mainly the AC part of the Doppler signal is amplified before digitalisation. This leads to signals that have less noise and with a smaller standard deviation and thus a higher sensitivity to physiological changes of perfusion parameters such as Vel and Vol.

## Claims

1. Method for processing an electrical signal provided by a laser Doppler flowmeter, comprising the steps of:
- detecting at least a portion of a light beam scattered by a flowing liquid upon which a coherent light beam from a laser Doppler flowmeter impinges with a detector;
- generating an electrical signal proportional to said detected portion of the scattered light beam;
- separating the DC and AC components of said electrical signal so as to provide a DC signal and an AC signal;
- amplifying the AC component by a first amplification and the DC component by a second amplification factor to obtain amplified AC and DC signals such that an absolute value of said amplified AC component, expressed in voltage, is comprised preferably between 0.5 and 100 times an absolute value, expressed in voltage, of said amplified DC component;
- providing a variable offset electrical signal;
- generating a weighted signal from said variable offset electrical signal, said amplified AC component and said amplified DC component;
- filtering said weighted signal;
- converting said filtered signal with an A/D converter and processing the converted signal with computing means to obtain a laser Doppler signal proportional to the liquid flow.

2. The method according to claim 1, wherein said amplified AC component has a value, expressed in voltage, between 0.5 and 50 times the value, expressed in voltage, of said amplified DC component.

3. The method according to claim 1 or claim 2, wherein said amplified AC component has a value, expressed in voltage, between 1 and 25 times the value, expressed in voltage, of said amplified DC component.

4. The method according to any one of claims 1 to 3, wherein said amplified DC component has a value, expressed in voltage, between 0.8 and 1.2 times the value, expressed in voltage, of said amplified AC component.

5. The method according to any one of claims 1 to 4, wherein said flowing liquid is a liquid flowing in a partially transparent tube.

6. The method according to claim 5, wherein said tube is an artery or a vein and said liquid is liquid blood.

7. The method according to claim 5, wherein said tube is a mesh of fluid capillaries of a living tissue.

8. The method according to claim 7, wherein said fluid capillaries are blood capillaries.

9. A laser Doppler flowmeter comprising a laser to send, in operation, a light beam onto a flowing liquid, and comprising optical elements to collect, in operation, backscattered light from said flowing liquid, further comprising a laser Doppler flowmetry acquisition system comprising a detector to collect said backscattered light comprising a DC and an AC signal component, **characterised in that** said laser Doppler flowmetry acquisition system comprises:
- means to separate the DC and AC signal component of said electrical signal so as to provide a DC signal and an AC signal;
- first amplification means to amplify said AC signal by a first amplification factor so as to obtain an amplified AC signal;
- second amplification means arranged to amplify said DC signal component by a second amplification factor so as to obtain an amplified DC signal and so that said amplified AC signal has a value, expressed in voltage, preferably between 0.5 and 100 times the value, expressed in voltage, of said amplified DC signal
- means arranged to provide a variable offset signal;
- a weighted summing amplifier arranged to input said variable offset, said amplified AC and said amplified DC component, so as to obtain, in operation, a weighted signal;
- a low-pass filter arranged to input said weighted signal, to provide a filtered signal;
- an A/D converter arranged to input said filtered signal, to provide a converted signal.

10. The laser Doppler flowmeter according to claim 9, wherein said first amplification factor is between 0.5 and 50.

11. The laser Doppler flowmeter according to claim 9, wherein said first amplification factor is between 1 and 25.

12. The laser Doppler flowmeter according to any one of claims 9 to 11, wherein said laser Doppler flowmetry acquisition system comprises an electronic circuit to perform a Fourier transform on said converted signal so as to provide a laser Doppler signal proportional to the flow of said liquid.

13. The laser Doppler flowmeter according to any one of claims 9 to 12, wherein said laser Doppler flowmetry acquisition system comprises computing means to process further the laser Doppler signal.

14. Use of a laser Doppler flowmeter according to any one of claims 9 to 13, for measuring blood flow in a blood vessel situated in a human or animal eye.

15. Use of a laser Doppler flowmeter according any one of claims 9 to 14, for measuring blood flow in a blood vessel situated in a human or animal skin.
